(19) Europäisches Patentamt
European Patent Office
Office européen des brevets

(11) Numéro de publication: **0 233 622 B1**

# (12) FASCICULE DE BREVET EUROPEEN

(45) Date de publication de fascicule du brevet: **26.06.91**

(51) Int. Cl.$^5$: **C07C 239/08**

(21) Numéro de dépôt: **87102147.3**

(22) Date de dépôt: **16.02.87**

## (54) Procédé pour la fabrication d'hydroxylamines.

(30) Priorité: **17.02.86 FR 8602217**

(43) Date de publication de la demande: **26.08.87 Bulletin 87/35**

(45) Mention de la délivrance du brevet: **26.06.91 Bulletin 91/26**

(84) Etats contractants désignés: **AT BE CH DE ES GB IT LI NL SE**

(56) Documents cités: **US-A- 3 243 462**

(73) Titulaire: **INTEROX Société Anonyme**
**Rue du Prince Albert, 33**
**B-1050 Bruxelles(BE)**

(72) Inventeur: **Legrand, Franz**
**Rue des Vaches, 71**
**B-7300 Quaregnon(BE)**
Inventeur: **Deschrijver, Paul**
**Jan Dekinderstraat, 63**
**B-1730 Asse-Zellik(BE)**

(74) Mandataire: **Keller, Günter et al**
**Lederer, Keller & Riederer Lucile-Grahn-Strasse 22**
**W-8000 München 80(DE)**

**EP 0 233 622 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention concerne un procédé pour la fabrication d'hydroxylamines N,N disubstituées selon lequel on oxyde une amine secondaire au moyen de peroxyde d'hydrogène dans un milieu solvant et en présence d'un catalyseur.

On connaît depuis longtemps un procédé de synthèse de la diméthylhydroxylamine par oxydation de la diméthylamine au moyen de peroxyde d'hydrogène en présence d'oxydes de Se, Mo, W, V, U et de dérivés de ces oxydes comme catalyseurs. Ces catalyseurs ne sont toutefois efficaces que pour l'oxydation de la seule diméthylhydroxylamine et les rendements obtenus restent cependant peu élevés (brevet US-A-3243462 (H.Q. SMITH et al.)* colonne 2, lignes 23 à 36 et lignes 65 à 67 et colonne 3, exemple 3 *).

Dans le brevet US-A-3467711 (H. BADER et al.), on enseigne que l'utilisation de catalyseurs d'oxydation tels que des tungstates ou des vanadates n'est pas adéquate et on préconise l'emploi exclusif d'agents séquestrants des métaux catalysant la décomposition du peroxyde d'hydrogène (* colonne 2, lignes 43 à 68 et colonnes 4 et 5, revendication 1 *). Les rendements obtenus en appliquant cette technique ne dépassent pas 55 % en hydroxylamine N,N disubstituée.

La présente invention vise à fournir un procédé pour la fabrication d'hydroxylamines N,N disubstituées qui ne présente pas les désavantages des procédés connus. Elle a en particulier pour objectif de permettre des rendements plus élevés en hydroxylamines N,N disubstituées. D'autres objectifs de l'invention sont une simplification de la séparation des hydroxylamines du milieu réactionnel et une réduction du coût en réactifs en n'imposant plus l'emploi de séquestrants d'ions métalliques.

A cet effet, l'invention concerne un procédé pour la fabrication d'hydroxylamines N,N disubstituées selon lequel on oxyde une amine secondaire au moyen de peroxyde d'hydrogène en présence d'un catalyseur; selon l'invention, on sélectionne le catalyseur parmi les composés du zinc et du cadmium.

Le catalyseur mis en oeuvre dans le procédé selon l'invention peut consister en un dérivé du zinc ou du cadmium. Parmi ces dérivés, les sels, les oxydes et les hydroxydes de zinc ou de cadmium conviennent bien. Des sels et des hydroxydes de zinc et de cadmium qui conviennent spécialement bien sont les halogénures et les carbonates, spécialement les chlorures et les hydroxycarbonates de zinc ou de cadmium.

Selon l'invention, le catalyseur mis en oeuvre peut aussi consister en un dérivé organique du zinc ou du cadmium. Les sels d'acides carboxyliques du zinc et du cadmium conviennent particulièrement bien.

Le catalyseur doit être mis en oeuvre en des concentrations d'au moins 0,02 mmole par mole d'amine à oxyder. En principe, il n'y a pas de limite supérieure à la quantité de catalyseur mise en oeuvre. Pour des raisons économiques il n'est pas conseillé, en pratique de dépasser la dose de 20 mmoles de catalyseur par mole d'amine. Les teneurs avantageuses sont celles comprises entre 0,2 et 10, préférablement entre 0,2 et 2,5 mmoles par mole d'amine. Le catalyseur peut être ajouté au milieu réactionnel à l'état pur, en solution dans l'eau ou dans un solvant ou en dispersion dans ces mêmes milieux.

L'invention s'applique à l'oxydation d'amines secondaires de types divers. Elle s'applique en particulier à l'oxydation des amines secondaires aliphatiques, des amines secondaires aromatiques et des amines secondaires cyclaniques. Les amines secondaires aliphatiques conviennent particulièrement bien, spécialement celles où les deux groupes aliphatiques liés à l'azote sont des groupes alkyle à chaîne droite ou ramifiée et plus particulièrement celles où chaque groupe aliphatique comprend 1-3 atomes de carbon. Ces groupes alkyle peuvent être identiques ou différents. Des exemples de telles amines qui conviennent particulièrement bien sont la N,N diméthylamine, la N,N diéthylamine, la N méthyl-,N éthylamine, la N,N di-n-propylamine, la N méthyl-,Nn-propylamine, la N éthyl-,Nn-propylamine, la N,N di-isopropylamine, la Nn-propyl-,N isopropylamine, la N méthyl-,N isopropylamine et la N éthyl-,N isopropylamine, l'énumération n'étant pas limitative.

Parmi les amines secondaires aromatiques que l'on peut oxyder suivant le procédé selon l'invention, sont avantageuses celles où le group aromatique est constitué d'un noyau benzénique non substitué telles que, par exemple, la N méthyl-,N phénylamine, la N éthyl-,N phénylamine, la Nn-propyl-,N phénylamine, la N isopropyl-,N phénylamine et la N,N diphénylamine.

Dans la classe des amines secondaires cyclaniques utilisables dans le procédé selon l'invention conviennent bien celles où le groupe cyclanique est constitué d'un groupe cyclohexane non substitué telles que, par exemple, la N méthyl-,N cyclohexylamine, la N éthyl-,N cyclohexylamine, la Nn-propyl,N cyclohexylamine, la N isopropyl,N cyclohexylamine et la N,N dicyclohexylamine.

L'invention porte aussi sur l'utilisation des amines secondaires des trois classes aliphatiques, aromatiques et cyclaniques qui portent des subtituants fonctionnels sur les groupes alkyle, aryle ou cycloakyle liés à l'azote. Parmi les substituants possibles conviennent, par exemple, les fonctions acide carboxylique, halogène, une autre fonction amine (primaire, secondaire ou tertiaire), éther, ester, oxime, nitro, nitrile,

sulfonate, cétone et aldéhyde.

L'examen de l'influence du rapport molaire amine/peroxyde d'hydrogène a montré que plus ce rapport est élevé, plus le rendement en hydroxylamine par rapport à la quantité de $H_2O_2$ mise en oeuvre est élevée, ce rendement étant défini par la relation :

$$\left( \frac{\text{nombre de moles d'hydroxylamine formées}}{\text{nombre de moles de } H_2O_2 \text{ mises en oeuvre}} \right) \times 100$$

Des rapports molaires amine/peroxyde d'hydrogène compris entre 1,5 et 10 conviennent bien. Les rapports molaires compris entre 2,5 et 4 sont préférés car ils conduisent à ne devoir recycler que l'amine, le taux de transformation de $H_2O_2$ :

$$\left( \frac{\text{nombre de moles de } H_2O_2 \text{ consommées}}{\text{nombre de moles de } H_2O_2 \text{ mises en oeuvre}} \right) \times 100$$

étant voisin de 100 %. En pratique, il est recommandable de maintenir le rapport molaire amine/peroxyde d'hydrogène aux environs de 2,8 pour ne pas devoir recycler une trop grande quantité d'amine.

Le peroxyde d'hydrogène peut être mis en oeuvre sous la forme d'une solution aqueuse commerciale ou sous la forme d'une solution dans un solvant organique. Les solutions aqueuses sont généralement préférées en raison de leur moindre coût et de leur plus grande disponibilité. Des solutions aqueuses contenant au moins 10 % en poids et pas plus de 95 % en poids de peroxyde d'hydrogène conviennent bien. De préférence, on emploie des solutions qui contiennent entre 20 et 85 % en poids de peroxyde d'hydrogène.

Dans le procédé selon l'invention, l'oxydation peut avantageusement être effectuée en milieu liquide dans un solvant inerte.

Il est généralement avantageux que le milieu liquide soit obtenu par dissolution dans un solvant inerte de l'amine secondaire, du peroxyde d'hydrogène et du catalyseur. Il n'est pas nécessaire que le catalyseur soit complètement dissous, sa présence à l'état dispersé dans le milieu liquide étant suffisante.

Le solvant inerte du procédé selon l'invention a pour fonction de réaliser un milieu liquide homogène capable, dans les conditions de la réaction, de solubiliser l'amine secondaire, le peroxyde d'hydrogène et l'hydroxylamine formée. Il doit être inerte vis-à-vis de l'amine, du peroxyde d'hydrogène et des produits de l'oxydation. Il peut être l'eau ou un solvant organique résistant à l'oxydation par le peroxyde d'hydrogène dans les conditions de la réaction. Les solvants organiques les plus avantageux sont ceux dont la constante diélectrique à 25 °C et à pression atmosphérique est supérieure à 15.

Dans une forme de réalisation préférée du procédé selon l'invention, le solvant sélectionné comprend de l'eau. Une partie de celle-ci peut être introduite avec les réactifs, en particulier le peroxyde d'hydrogène; enfin, il s'en forme également pendant l'oxydation de l'amine.

Dans une variante de cette forme de réalisation, pour faciliter la dissolution de l'amine, du peroxyde d'hydrogène et de l'hydroxylamine, on mélange un ou plusieurs solvants organiques. Parmi les solvants qui conviennent bien, on trouve, par exemple, les alcools aliphatiques inférieurs éventuellement substitués tels que le méthanol, l'éthanol, l'isopropanol, le n-butanol et le trifluoroéthanol, les glycols tels que l'éthylèneglycol, les 1,3-butanediol, le 1,4-butanediol et 2,3-butanediol, le glycérol, les éthers tels que le diéthyléther et le 1,4-dioxane et les amides telles que la formamide, l'acétamide et la N,N-diméthylformamide.

On a constaté qu'en plus de sa fonction de solvant polaire, l'eau exerce une influence favorable sur le rendement en hydroxylamine par rapport au peroxyde d'hydrogène et qu'il était par conséquent avantageux de maintenir dans le milieu réactionnel une quantité non négligeable d'eau et, avantageusement supérieure à 10 % en poids du mélange réactionnel.

Dans un mode d'exécution particulier de cette variante du procédé selon l'invention, on réalise l'oxydation catalytique de l'amine secondaire dans un système biphasique eau/phase organique au moyen de la technique de catalyse par transfert de phases. Cette technique est particulièrement avantageuse lorsque le catalyseur ou l'amine est quasi insoluble dans l'eau.

La température et la pression auxquelles on effectue la réaction d'oxydation selon l'invention peuvent varier dans de larges limites. Sans être critiques, on observe cependant, particulièrement dans le cas de la température, une plage de conditions optimales qui dépendent de la nature de l'amine secondaire, de sa concentration et de celle du peroxyde d'hydrogène, de la composition du solvant et des autres conditions opératoires en général. Les températures et pressions optimales doivent être déterminées soigneusement dans chaque cas particulier envisagé. Cette détermination peut se faire en laboratoire à l'aide d'essais de synthèses de routine.

La durée de la réaction d'oxydation dépend de la nature de l'amine à oxyder ainsi que du catalyseur et du solvant mis en oeuvre. Elle peut varier entre 10 et 90 minutes. De préférence, la durée de réaction est supérieure à 30 minutes et ne dépasse pas 75 minutes.

Le procédé selon l'invention peut être mis en oeuvre en continu ou en discontinu, dans un réacteur unique ou dans une batterie de réacteurs disposés en parallèle ou en série. Pour réaliser le procédé selon l'invention, on peut utiliser n'importe quel appareillage convenant pour les mélanges réactionnels liquides.

Le catalyseur et les réactifs peuvent être introduits de diverses manières connues en soi. On peut ainsi procéder à une introduction unique, à une introduction continue ou à une introduction étagée du catalyseur, de l'amine secondaire et/ou du peroxyde d'hydrogène.

Un mode de réalisation particulièrement préféré de l'invention consiste à mélanger au préalable le solvant, le catalyseur et l'amine secondaire, puis à introduire le peroxyde d'hydrogène dans le mélange sous agitation en un temps relativement court n'excédant pas 20 minutes et de préférence de l'ordre de 5 à 10 minutes.

Après réaction, le milieu réactionnel peut être soumis à diverses techniques de séparation telles que la distillation et la séparation pour recueillir l'hydroxylamine et les réactifs non transformés que l'on peut avantageusement recycler au procédé.

Lorsque le procédé selon l'invention est réalisé en continu, on peut avantageusement utiliser l'appareil décrit et illustré dans le brevet français FR-B-8112797 déposé le 26 juin 1981 (INTEROX - Société Anonyme).

Les hydroxylamines N,N disubstituées fabriquées au moyen du procédé selon l'invention peuvent être utilisés comme produits intermédiaires dans les synthèses organiques diverses. Elles trouvent également des usages comme inhibiteurs de polymérisation des oléfines dans l'industrie du caoutchouc grâce à leur propriété de capter les radicaux libres, comme inhibiteur d'oxygène dans les chaudières, comme produit pharmaceutique et agent de stabilisation du styrène.

Des particularités de l'invention ressortiront des exemples suivants qui décrivent des procédés de fabrication d'hydroxylamines N,N disubstituées conformes à l'invention ainsi que des essais de comparaison avec des procédés connus.

Exemple 1R (procédé connu de référence)

Dans un réacteur en verre de 500 ml à double enveloppe permettant le chauffage par circulation d'huile et muni d'un serpentin de refroidissement, on a introduit 40 ml (0,387 mole) de diéthylamine et 38 ml d'eau à température ambiante via une ouverture d'alimentation située dans la partie supérieure du réacteur. Après avoir refermé le réacteur, on a amené la température du milieu réactionnel à 60 °C puis on a introduit 12 ml d'une solution aqueuse à 40 % en poids de peroxyde d'hydrogène (0,20 mole) de manière continue pendant 6 minutes. On a ensuite laissé réagir pendant 45 minutes puis on a coupé le chauffage de la double enveloppe et refroidi rapidement le milieu au moyen d'eau fraîche que l'on a fait circuler dans le serpentin de refroidissement.

Lorsque le liquide réactionnel a été ramené à température ambiante, on a introduit 10 ml de dioxane à titre de standard interne pour l'analyse chromatographique, puis on a homogénéisé l'ensemble.

On a ensuite procédé à l'analyse du liquide réactionnel au moyen de la technique de chromatographie en phase vapeur (colonne capillaire en silice fondue, gaz porteur hélium). On a dosé 0,168 mole de N,N diéthylhydroxylamine dans le milieu, ce qui correspond à un rendement en N,N diéthylhydroxylamine par rapport à l'$H_2O_2$ mise en oeuvre de 56,6 %.

Exemple 2R (procédé connu de référence)

On a procédé suivant le même mode opératoire que dans l'exemple 1R excepté que le mélange des réactifs a été additionné de 0,329 g $Na_2WO_4$ à titre de catalyseur.

On a dosé 0,012 mole de N,N diéthylhydroxylamine dans les produits de la réaction, soit un rendement par rapport à l'$H_2O_2$ mise en oeuvre de 6,0 %.

Exemple 3 (conforme à l'invention)

On a suivi le même mode opératoire que dans l'exemple 2R, excepté pour le catalyseur qui a consisté ici en 0,021 g de $CdCl_2.2H_2O$ (0,1 mmol de Cd).

On a dosé 0,134 mole de N,N diéthylhydroxylamine dans le milieu réactionnel, soit un rendement par rapport à l'$H_2O_2$ mise en oeuvre de 67,0 %.

Cet exemple comparé aux exemples 1R et 2R illustre l'efficacité du catalyseur selon l'invention.

Exemples 4 à 15 (conformes à l'invention)

Les exemples 4 à 15 ont été effectués selon l'invention à l'intervention de catalyseurs à base de zinc et ont eu pour objectif d'examiner l'influence de la quantité de catalyseur.

Le mode opératoire suivi a été pour tous ces essais, semblable à celui de l'exemple 2R.

Le résultat des analyses chromatographiques a permis de calculer les rendements en hydroxylamine produite par rapport à la quantité d'$H_2O_2$ mise en oeuvre

$$(R_{H_2O_2}).$$

Ces rendements ont été portés au tableau I qui suit.

Tableau I

| N° Exemple | Nature du catalyseur | Catalyseur mis en oeuvre, mmole Zn | $R_{H_2O_2}$ % |
|---|---|---|---|
| 4 | $ZnCl_2$ | 2,05 | 70 |
| 5 | | 1,36 | 73 |
| 6 | | 0,68 | 70 |
| 7 | | 0,34 | 69 |
| 8 | | 0,15 | 66 |
| 9 | | 0,03 | 67 |
| 10 | $2ZnCO_3 . 3Zn(OH)_2$ | 1,37 | 75 |
| 11 | | 0,46 | 74 |
| 12 | | 0,05 | 69 |
| 13 | $Zn(CH_3-COO)_2 . 2H_2O$ | 1,37 | 77 |
| 14 | | 0,68 | 70 |
| 15 | | 0,34 | 68 |

Les rendements obtenus montrent l'efficacité remarquable du catalyseur même lorsque sa concentration dans le milieu est très faible. D'autre part, ils indiquent une performance sensiblement analogue pour les dérivés inorganiques ou organiques du zinc.

Exemples 16 à 20 (conformes à l'invention)

Ces exemples ont eu pour but d'étudier l'influence du rapport molaire amine/$H_2O_2$ sur le rendement

$$R_{H_2O_2}$$

défini ci-dessus. Ces exemples ont été effectués dans le même appareillage et suivant la même procédure qu'à l'essai 1R. Les conditions particulières des essais 16 à 20 ont été les suivantes :

| | |
|---|---|
| Solvant : $H_2O$ | 50 ml |
| Amine : $(CH_3-CH_2)_2-NH$ | 0,852 mole |
| $H_2O_2$ : 84 % en poids | quantité variable suivant l'essai |
| Catalyseur : $2ZnCO_3 \cdot 3Zn(OH)_2$ | 2,2 mmoles de Zn |
| Temps d'introduction de $H_2O_2$ | 15 min. |
| Température de réaction | 60°C |
| Temps de réaction | 60 min. |

Les rendements déterminés par chromatographie en phase vapeur figurent au tableau II.

Tableau II

| N° Exemple | Rapport molaire amine/$H_2O_2$ | $R_{H_2O_2}$ % |
|---|---|---|
| 16 | 1,75 | 63 |
| 17 | 2,20 | 71 |
| 18 | 2,90 | 75 |
| 19 | 4,40 | 80 |
| 20 | 8,80 | 86 |

Exemples 21 à 23 (conformes à l'invention)

Dans les exemples 21 à 23, tous conformes à l'invention, on a cherché à étudier l'incidence de la température de réaction sur le rendement

$$R_{H_2O_2}$$

en N,N diéthylhydroxylamine.

Les conditions particulières des essais 21 à 23 ont été celles qui suivent :

| | |
|---|---|
| Solvant : $H_2O_2$ | 30 ml |
| Amine : $(CH_3-CH_2)_2-NH$ | 0,852 mole |
| $H_2O_2$ : 84 % en poids | 0,300 mole |
| Catalyseur : $2ZnCO_3 \cdot 3Zn(OH)_2$ | 2,25 mmoles de Zn |
| Temps d'introduction de $H_2O_2$ | 45 min. |
| Temps de réaction | 60 min. |

Les résultats obtenus ont été portés au tableau III.

**Tableau III**

| N° Exemple | Température de réaction °C | $R_{H_2O_2}$ % |
|---|---|---|
| 21 | 50 | 35 |
| 22 | 60 | 71 |
| 23 | 70 | 65 |

Exemples 24 à 28 (conformes à l'invention)

Ces exemples ont été effectués pour étudier l'influence du temps de réaction sur le rendement

$$R_{H_2O_2}$$

en N,N diéthylhydroxylamine.

Les conditions particulières à ces exemples ont été les suivantes :

| | |
|---|---|
| Solvant : $H_2O_2$ | 50 ml |
| Amine : $(CH_3-CH_2)_2-NH$ | 0,852 mole |
| $H_2O_2$ : 84 % en poids | 0,300 mole |
| Catalyseur : $2ZnCO_3 \cdot 3Zn(OH)_2$ | 2,25 mmoles de Zn |
| Temps d'introduction de $H_2O_2$ | 6 min. |
| Température de réaction | 60°C |

Les résultats obtenus figurent au tableau IV.

Tableau IV

| N° Exemple | Temps de réaction, min. | $R_{H_2O_2}$ % |
|---|---|---|
| 24 | 15 | 23,5 |
| 25 | 30 | 73,5 |
| 26 | 45 | 75,0 |
| 27 | 60 | 75,05 |
| 28 | 90 | 75,1 |

Exemples 29 R (procédé connu de référence)

Cet exemple illustre la synthèse de di-n-propylhydroxylamine par le procédé connu d'oxydation au moyen de peroxyde d'hydrogène en l'absence de catalyseur.

Le mode opératoire a été semblable à celui utilisé à l'exemple 1R. Les conditions particulières à l'exemple 29 R ont toutefois été :

| | |
|---|---|
| Solvant : $CH_3OH$ | 60 ml |
| Amine : $(CH_3-CH_2-CH_2)_2-NH$ | 0,718 mole |
| $H_2O_2$ : 84 % en poids | 0,264 mole |
| Temps d'introduction de $H_2O_2$ | 10 min. |
| Température de réaction | 75°C |
| Standard interne introduit pour analyse CPV : | Cyclohexanol, 10 g |

L'analyse du milieu de réaction par la technique de chromatographie en phase vapeur a indiqué qu'un rendement

$$R_{H_2O_2}$$

en N,N di-n-propylhydroxylamine de 8,7 % par rapport au peroxyde d'hydrogène mis en oeuvre.

Exemples 30 à 33 (conformes à l'invention)

Ces exemples ont été effectués en vue d'étudier l'influence du temps de réaction sur le rendement

$$R_{H_2O_2}$$

en N,N di-n-propylhydroxylamine.

Le mode opératoire utilisé a été celui de l'exemple 2R. Les conditions particulières aux exemples 30 à 33 ont été :

| | |
|---|---|
| Solvant : $CH_3OH$ | 60 ml |
| Amine : $(CH_3-CH_2-CH_2)_2-NH$ | 0,718 mole |
| $H_2O_2$ : 84 % en poids | 0,264 mole |
| Catalyseur : $2ZnCO_3 . 3Zn(OH)_2$ | 0,46 mmole de Zn |
| Temps d'introduction de $H_2O_2$ | 10 min. |
| Température de réaction | 75°C |

Les résultats ont été portés au tableau V.

Tableau V

| N° Exemple | Temps de réaction, min. | $R_{H_2O_2}$ % |
|---|---|---|
| 34 | 30 | 53,0 |
| 35 | 45 | 59,0 |
| 36 | 60 | 62,0 |
| 37 | 75 | 63,5 |

**Revendications**

1. Procédé pour la fabrication d'hydroxylamines N,N disubstituées selon lequel on oxyde une amine secondaire au moyen de peroxyde d'hydrogène en présence d'un catalyseur, caractérisé en ce que le catalyseur est sélectionné parmi les composés du zinc et du cadmium.

2. Procédé suivant la revendication 1, caractérisé en ce que le catalyseur est sélectionné parmi les sels et les hydroxydes de zinc et de cadmium.

3. Procédé suivant la revendication 2, caractérisé en ce que le catalyseur est un chlorure, un acétate ou un hydroxycarbonate de zinc et de cadmium.

4. Procédé suivant l'une quelconque des revendications 1 à 3, caractérisé en ce que l'oxydation est opérée dans un milieu solvant inerte choisi parmi l'eau et les solvants organiques dont la constante diélectrique est supérieure à 15 à pression atmosphérique et à température de 25° C.

5. Procédé suivant l'une quelconque des revendications 1 à 4, caractérisé en ce que l'amine secondaire est sélectionnée parmi les amines appartenant aux classes des amines aliphatiques et des amines aromatiques.

6. Procédé suivant l'une quelconque des revendications 1 à 5, caractérisé en ce que le peroxyde d'hydrogène mis en oeuvre est une solution aqueuse contenant plus de 10 % en poids et moins de 95 % en poids de peroxyde d'hydrogène.

7. Procédé suivant l'une quelconque des revendications 1 à 6, caractérisé en ce que le catalyseur est utilisé en une quantité comprise entre 0,02 et 10 millimoles par mole d'amine mise en oeuvre.

8. Procédé suivant l'une quelconque des revendications 1 à 7, caractérisé en ce qu'on maintient le rapport molaire amine/peroxyde d'hydrogène entre 1,5 et 10.

9. Procédé suivant l'une quelconque des revendications 1 à 8, caractérisé en ce qu'on effectue la réaction à une température comprise entre 50 et 80° C à pression atmosphérique pendant une durée comprise entre 30 et 75 minutes.

## Claims

1. A process for the production of N,N-disubstituted hydroxyl amines according to which a secondary amine is oxydized by means of hydrogen peroxide in the presence of a catalyst, characterised in that the catalyst is selected from the compounds of zinc and cadmium.

2. A process according to claim 1, characterised in that the catalyst is selected from the salts of the hydroxides of zinc and cadmium.

3. A process according to claim 2, characterised in that the catalyst is a chloride, an acetate or a hydroxy carbonate of zinc or cadmium.

4. A process according to any one of claims 1 to 3, characterised in that the oxidation is carried out in an inert solvent atmosphere selected from water and the organic solvents whose dielectric constant is higher than 15 at atmospheric pressure and at a temperature of 25° C.

5. A process according to any one of claims 1 to 4, characterised in that the secondary amine is selected from the amines belonging to the groups of aliphatic amines and aromatic amines.

6. A process according to any one of claims 1 to 5, characterised in that the hydrogen peroxide applied is an aqueous solution containing more than 10 % by weight and less than 95 % by weight of hydrogen peroxide.

7. A process according to any one of claims 1 to 6, characterised in that the catalyst is applied in a quantity comprising between 0.02 and 10 millimol per mol of the used amine.

8. A process according to any one of claims 1 to 7, characterised in that the molar ratio of amine / hydrogen peroxide is maintained between 1.5 and 10.

9. A process according to any one of claims 1 to 8, characterised in that the reaction takes place at a temperature comprised between 50 and 80° C at atmospheric pressure during a period of time of between 30 and 75 minutes.

## Ansprüche

1. Verfahren zur Herstellung von N,N-disubstituierten Hydroxylaminen gemäß dem ein sekundäres Amin

oxidiert wird mit Hilfe von Wasserstoffperoxid in Anwesenheit eines Katalysators, dadurch gekennzeichnet, daß der Katalysator ausgewählt ist aus den Verbindungen von Zink und Kadmium.

2. Verfahren nach Anspruch 1, dadurch gekennzeichnet, daß der Katalysator ausgewählt ist aus den Salzen und den Hydroxiden von Zink und Cadmium.

3. Verfahren nach Anspruch 2, dadurch gekennzeichnet, daß der Katalysator ein Chlorid, ein Acetat oder ein Hydroxycarbonat von Zink und Kadmium ist.

4. Verfahren nach einem der Ansprüche 1 bis 3, dadurch gekennzeichnet, daß die Oxidation in einer inerten Lösungsmittelumgebung ausgewählt aus Wasser und den organischen Lösungsmitteln, deren Dielektrizitätskonstante höher ist als 15 bei atmosphärischem Druck und bei einer Temperatur von 25° C, durchgeführt wird.

5. Verfahren nach einem der Ansprüche 1 bis 4, dadurch gekennzeichnet, daß das sekundäre Amin ausgewählt ist aus den Aminen, die den Klassen der aliphatischen Amine und der aromatischen Amine angehören.

6. Verfahren nach einem der Ansprüche 1 bis 5, dadurch gekennzeichnet, daß das eingesetzte Wasserstoffperoxid eine wäßrige Lösung ist, die mehr als 10 Gewichts-% und weniger als 95 Gewichts-% Wasserstoffperoxid enthält.

7. Verfahren nach einem der Ansprüche 1 bis 6, dadurch gekennzeichnet, daß der Katalysator in einer Menge umfassend zwischen 0,02 und 10 Millimol je Mol des eingesetzten Amins verwendet wird.

8. Verfahren nach einem der Ansprüche 1 bis 7, dadurch gekennzeichnet, daß das molare Verhältnis von Amin : Wasserstoffperoxid zwischen 1,5 und 10 eingehalten wird.

9. Verfahren nach einem der Ansprüche 1 bis 8, dadurch gekennzeichnet, daß die Umsetzung bei einer Temperatur von zwischen 50° und 80° C bei atmosphärischem Druck in einem Zeitraum zwischen 30 und 75 Minuten bewirkt wird.